# EUROPÄISCHE PATENTSCHRIFT

(11) **EP 2 661 287 B1**
(45) Veröffentlichungstag und Bekanntmachung des Hinweises auf die Patenterteilung: **14.01.2015**
(21) Anmeldenummer: 11801604.7
(22) Anmeldetag: 15.12.2011
(51) Int. Cl.: A61M 1/00

(54) **ABSAUGVORRICHTUNG**
SUCTION DEVICE
DISPOSITIF D'ASPIRATION

(30) Priorität: 07.01.2011 DE 102011008051
(43) Veröffentlichungstag der Anmeldung: 13.11.2013
(73) Patentinhaber: Georg-August-Universität Göttingen - Stiftung Öffentlichen Rechts/Universitätsmedizin, 37075 Göttingen (DE)
(72) Erfinder: FRIEDRICH, Martin, 37120 Bovenden (DE)
(74) Vertreter: Günther, Constantin
(86) Internationale Anmeldenummer: PCT/EP2011/006330
(87) Internationale Veröffentlichungsnummer: WO 2012/092948

(56) Entgegenhaltungen:
- WO-A2-2007/103279
- WO-A2-2010/075502
- DE-A1- 3 418 341
- US-A- 4 976 682
- US-A1- 2010 211 029

## Beschreibung

Die Erfindung betrifft eine Absaugvorrichtung zur Absaugung von Flüssigkeiten, mit einem zur Aufnahme der Flüssigkeit mit wenigstens einer Saugöffnung versehenen Sauger und einer mit dem Sauger verbundenen Pumpe, die dazu eingerichtet ist, im Sauger einen Saug-Unterdruck zu erzeugen, wobei eine Steuereinrichtung und wenigstens ein mit der Steuereinrichtung verbundener Sensor vorgesehenen ist, wobei die Steuereinrichtung dazu eingerichtet ist, in Abhängigkeit von den von dem Sensor aufgenommenen Signalen die an der Saugöffnung wirksame Saugleistung zu beeinflussen.

Eine solche Absaugvorrichtung kann sowohl im medizinischen als auch im nicht-medizinischen Bereich verwendet werden. Es werden daher Anwendungsmöglichkeiten im medizinischen Bereich in Form von medizinischen Absaugvorrichtungen und Anwendungen im nicht-medizinischen Bereich erläutert.

Derartige medizinische Absaugvorrichtungen werden, z. B. in Form von chirurgischen Saugern, zum Sammeln von Blut und anderen Körperflüssigkeiten während einer chirurgischen Operation verwendet. Das Blut wird nach entsprechender Aufbereitung wieder dem Körper eines Patienten, dem es entnommen wurde, zugeführt. Hierbei ist es von Bedeutung, dass das Blut schonend abgesaugt und aufbereitet wird, um eine damit einhergehende globale Schädigung aller Blutzellen (insbesondere rote Blutkörperchen (Hämolyse) und Blutplättchen (Thrombozythopathie, Thrombozytopenie)) und auch anderer Blutbestandteile (Eiweißkörper, Botenstoffe) soweit wie möglich zu vermeiden. Dem Stand der Technik sind Vorschläge für eine automatische Steuerung einer medizinischen Absaugvorrichtung zu entnehmen, die sich dadurch charakterisieren lassen, dass mittels eines Sensors ein leer laufender Sauger erkannt wird. Gemäß US 4,416,658 wird hierzu ein Drucksensor verwendet, gemäß DE 196 46 410 C2 ein optischer Sensor, gemäß DE 699 23 711 T2 ein Luftsensor zur Feststellung der Anwesenheit und der Menge von Luft in der Saugleitung und gemäß DE 34 18 341 ein Drucksensor oder ein Stromsensor. Weitere Absaugvorrichtungen sind bekannt aus der WO 2010/075502 A2, der WO 2007/103279 A2, der US 4,976,682, der US 2010/0211029 A1 und der DE 3418341 A1.

In der Praxis hat sich jedoch gezeigt, dass mit den bekannten Vorschlägen die in der chirurgischen Praxis auftretenden Situationen nicht umfassend abdeckbar sind. Bisher ist es trotzdem erforderlich, dass eine Bedienperson, die den Sauger führt, eine manuelle Beeinflussung der wirksamen Saugleistung vornimmt bzw. den Sauger immer wieder erneut günstig platziert.

Auch im nicht-medizinischen Bereich ist es erwünscht, die Absaugvorrichtung möglichst effektiv zu betreiben und beim Absaugen von Flüssigkeiten ein unerwünschtes Vermengen mit Luft aus der Umgebung zu vermeiden.

Der Erfindung liegt daher die Aufgabe zugrunde, eine Absaugvorrichtung zur Absaugung von Flüssigkeiten anzugeben, die mit höherer Effizienz als bekannte Absaugvorrichtungen arbeitet. Für den medizinischen Bereich besteht zudem ein Bedarf daran, eine medizinische Absaugvorrichtung anzugeben, die bei automatischem Betrieb die auftretende Schädigung von Blutbestandteilen weitestgehend vermeidet.

Diese Aufgabe wird durch eine Absaugvorrichtung mit den Merkmalen des Anspruchs 1 gelöst. Gemäß der Erfindung ist der Sensor als Schallwellensensor ausgebildet. Der Schallwellensensor ist dazu eingerichtet, durch den Sauger bei dessen Betrieb erzeugte Schallwellen aufzunehmen. Der Begriff Schallwellen umfasst dabei akustische Schwingungen und Vibrationen aller Art, wie z.B. Geräusche. Vorteilhafte Ausgestaltungen der Erfindung sind in den Unteransprüchen angegeben.

Nachfolgend werden Vorteile, Anwendungsmöglichkeiten und Weiterbildungen der Erfindung sowohl für den medizinischen Bereich als auch für den nicht-medizinischen Bereich erläutert. Zunächst sei auf den medizinischen Bereich eingegangen.

Gemäß der Erfindung werden durch den Schallwellensensor Schallwellen aufgenommen, die durch den Sauger erzeugt werden. In der chirurgischen Praxis hat es sich nämlich gezeigt, dass Betriebszustände des Saugers, in denen es vermehrt zu der eingangs erwähnten Schädigung der Blutzellen, insbesondere zu einer Hämolyse, kommt, durch die bei einem chirurgischen Eingriff anwesenden Personen ohne weiteres akustisch erkannt werden können, und zwar anhand besonderer, charakteristischer Geräusche, z. B. eines besonders scharf klingenden, schlürfenden Sauggeräuschs. Ein solches Geräusch kann durch einen Schallwellensensor und eine damit verbundene Steuereinrichtung detektiert werden. Als Schallwellensensor kann z. B. ein Messmikrophon bzw. ein Mikrophon mit Richtcharakteristik, das in Richtung zu der Saugöffnung ausgerichtet ist, verwendet werden.

Hiermit kann eine Sauger-Steuerung realisiert werden, die die blutschädigenden Turbulenzen beim Absaugen vermeidet, indem das typische schlürfende Sauggeräusch vermieden wird. Die Erfindung hat den weiteren Vorteil, dass für die Bedienung der Absaugvorrichtung während einer Operation nicht permanent ein hierfür bisher erforderlicher zweiter Assistent durchgehend zur Verfügung stehen muss. Vielmehr kann die Absaugvorrichtung weitgehend automatisch betrieben werden.

Der Sauger kann eine Saugöffnung oder mehrere Saugöffnungen aufweisen, z. B. eine oder mehrere frontseitige Saugöffnungen und eine oder mehrere seitliche Saugöffnungen. Als Schallwellensensor kommen alle Sensoren in Frage, die eine Erfassung von akustischen Schwingungen und Vibrationen aller Art erlauben.

Gemäß einer vorteilhaften Weiterbildung der Erfindung sind die Steuereinrichtung und/oder der Schallwellensensor dazu eingerichtet, anhand der aufgenommenen Schallwellen wenigstens ein vorbestimmtes charakteristisches Schallwellenmuster zu detektieren und bei Detektion eines solchen charakteristischen Schallwellenmusters die an der Saugöffnung wirksame Saugleistung zu verändern. Die Funktion zur Detektion des charakteristischen Schallwellenmusters kann in der Steuereinrichtung oder dem Schallwellensensor oder verteilt in beiden Einrichtungen vorgesehen sein, z. B. anhand von Frequenzfiltern und/oder einer elektronischen Signalverarbeitung der aufgenommenen Schallwellen. Hierfür können bestimmte Schallwellenmuster, die sich bei schlürfenden Sauggeräuschen typischerweise ergeben, als Vergleichsmuster z.B. in der Steuereinrichtung gespeichert sein. Die Vergleichsmuster werden dann mit den aufgenommenen Schallwellen verglichen. Bei einem bestimmten Übereinstimmungsgrad wird dann ein charakteristisches Schallwellenmuster als detektiert angesehen.

Gemäß einer vorteilhaften Weiterbildung der Erfindung ist die Steuereinrichtung dazu eingerichtet, bei Detektion eines solchen charakteristischen Schallwellenmusters die an der Saugöffnung wirksame Saugleistung zu reduzieren. Dies hat den Vorteil, dass die Körperflüssigkeit weniger schnell zur Saugöffnung und in den Sauger gesaugt wird, so dass insbesondere die roten Blutkörperchen weniger heftig auf Grenzflächen auftreffen. Hierdurch kann die Hämolyse deutlich reduziert werden. Weitere Vorteile sind das Vermeiden von Verwirbelungen, Turbulenzen und Beschleunigungen an Grenzstrukturen sowie eine Verringerung auftretender Scheerkräfte. Vorteilhaft ist zudem, dass ein Vermischen der abgesaugten Flüssigkeiten mit Luft vermieden oder zumindest verringert wird. Hierdurch können eine Schaumbildung reduziert und unerwünschte Effekte durch unterschiedliche Oberflächenspannungen vermindert werden.

Der Schallwellensensor kann grundsätzlich an jeder geeigneten Stelle platziert werden, wobei natürlich darauf zu achten ist, dass die Entfernung zum Sauger nicht zu groß ist, so dass die zu detektierenden charakteristischen Schallwellenmuster sicher erfasst werden können. Möglich ist z. B., ein Messmikrophon separat auf einem Stativ neben dem Operationstisch anzuordnen. Gemäß einer vorteilhaften Weiterbildung der Erfindung ist der Schallwellensensor am Sauger, an der Saugseite der Pumpe oder einer Verbindung zwischen dem Sauger und der Pumpe angeordnet. Der Schallwellensensor kann auch im Sauger, in der Pumpe oder in der Verbindung zwischen Sauger und Pumpe angeordnet sein. Hierdurch ist der Schallwellensensor in einem bestimmten vorgegebenen Bezug zu der Absaugvorrichtung angeordnet, die eine hinsichtlich der Erfassung der charakteristischen Schallwellenmuster günstige Platzierung ermöglicht. Insbesondere ist der Schallwellensensor nicht separat und lose von der Absaugvorrichtung vorgesehen, sondern damit in einer vorgegebenen und daher definierten Weise fest oder lösbar verbunden. Insbesondere kann der Schallwellensensor fest an den zuvor genannten Elementen der Absaugvorrichtung, insbesondere am Sauger, befestigt sein. Vorteilhaft ist auch eine Befestigung des Schallwellensensors mittels einer lösbaren Steckbefestigung. Hierbei kann der Schallwellensensor vorteilhaft an dem Sauger mittels der Steckbefestigung lösbar befestigbar ausgebildet sein.

Gemäß einer vorteilhaften Weiterbildung der Erfindung werden als charakteristische Schallwellenmuster Schallwellen im Frequenzbereich oberhalb von 11 kHz detektiert. Vorteilhaft ist es ebenfalls, als charakteristische Schallwellenmuster Schallwellen im Frequenzbereich oberhalb von 15 kHz zu detektieren. Gemäß einer vorteilhaften Weiterbildung der Erfindung werden als charakteristische Schallwellenmuster Schallwellen in einem Frequenzbereich bis maximal 50 kHz detektiert. Es hat sich gezeigt, dass durch die Erkennung von Schallwellen in diesen Frequenzbereichen eine zuverlässige Separierung und Detektion der Schlürfgeräusche möglich ist, auch bei Neben- und Hintergrundgeräuschen. Zur Detektion der Schallwellen in den genannten Frequenzbereichen kann z. B. ein Hochpassfilter oder ein Bandpassfilter verwendet werden.

Gemäß einer vorteilhaften Weiterbildung der Erfindung ist der Schallwellensensor als piezoelektrischer Sensor ausgebildet, der am Sauger befestigt ist. Es hat sich gezeigt, dass hierdurch auf einfache und kostengünstige Weise eine zuverlässige Detektion der charakteristischen Schlürfgeräusche realisiert werden kann. Durch den piezoelektrischen Sensor werden Schwingungen des Saugers direkt erfasst. Gemäß einer vorteilhaften Weiterbildung der Erfindung ist der piezoelektrische Sensor wenigstens zum Teil frei schwingend am Sauger befestigt. Dies ermöglicht eine weiter verbesserte Erfassung der Schallwellen. Durch die teilweise frei schwingende Befestigung des piezoelektrischen Sensors kann dieser zu stärkeren Schwingungen angeregt werden als bei vollflächiger Befestigung an dem Sauger. Hierdurch ist die Erfassungsempfindlichkeit zur Erfassung der charakteristischen Schallwellenmuster verbessert.

Gemäß einer vorteilhaften Weiterbildung der Erfindung können die Steuereinrichtung und/oder der Schallwellensensor mit einer Blutverarbeitungseinrichtung verbunden sein, die zur Aufbereitung des Blutes für die Rückführung in den Körper des Patienten vorgesehen ist. Die Blutverarbeitungseinrichtung ist dazu eingerichtet, die von dem Schallwellensensor aufgenommenen Schallwellen bzw. die detektierten charakteristischen Schallwellenmuster bei der Bestimmung einer Hämolyse-Punktzahl einzubeziehen. Mittels der Hämolyse-Punktzahl wird in der Blutverarbeitungseinrichtung die Blutaufbereitung so gesteuert, dass die Hämolyse weitgehend kompensiert wird, z. B. durch Abscheiden von Zelltrümmern, Fettpartikeln oder Gerinnseln. Gemäß einer vorteilhaften Weiterbildung gehen in die Hämolyse-Punktzahl die Laufzeit einer Herz-Lungen-Maschine und die summierten Zeiten, während denen charakteristische Schallwellenmuster detektiert wurden, ein. Die Zeiten, in denen ein Schlürfen des Saugers erkannt wurde, beeinflussen somit direkt die Blutverarbeitung. Die charakteristischen Schallwellenmuster können z. B. in Form eines zeitlichen Integrals eingehen, bei dem auch die Amplitude der Schallwellen berücksichtigt wird.

Eine weitere vorteilhafte Weiterbildung der Erfindung betrifft den Fall, dass sich der Sauger mehr oder weniger festsaugt. Dies stellt insbesondere beim Saugen und Entlasten des linken Ventrikels ein besonderes Problem dar.

Gemäß einer vorteilhaften Weiterbildung der Erfindung ist wenigstens ein Festsaug-Sensor vorgesehen, der derart mit dem Sauger, der Pumpe oder einer Verbindung dazwischen verbunden ist, dass ein Festsaugen des Saugers erfassbar ist. Als Festsaugen wird ein völliges oder weitgehendes Verstopfen oder Verschließen des Saugers verstanden, z.B. durch feste Partikel in der Flüssigkeit oder durch andere feste oder zähe Elemente in der Umgebung, so dass ein weiteres Absaugen von Flüssigkeit zumindest zu einem großen Teil verhindert ist. Eine solche automatische Erkennung des Festsaugens des Saugers erlaubt es ebenfalls automatisch Gegenmaßnahmen zu ergreifen, wie nachfolgend noch beschrieben wird.

Es sei darauf hingewiesen, dass eine Absaugvorrichtung mit den Merkmalen des Oberbegriffs des Anspruchs 1 auch für sich genommen eine Erfindung ist, wenn dabei wenigstens ein Festsaug-Sensor vorgesehen ist, der derart mit dem Sauger, der Pumpe oder einer Verbindung dazwischen verbunden ist, dass ein Festsaugen des Saugers erfassbar ist. Das Gleiche gilt für die nachfolgend genannten Weiterbildungen.

Das Festsaugen kann anhand des Signals des Festsaug-Sensors erkannt werden, z.B. anhand bestimmter charakteristischer Signalwerte oder anhand einer charakteristischen zeitlichen Kennlinie des Signals, die jeweils charakteristisch für das Festsaugen sind. So kann z.B. eine besondere Dynamik des Signals im zeitlichen Ablauf eines Festsaugevorgangs einem Erkennungsalgorithmus zugrunde gelegt werden.

Gemäß einer vorteilhaften Weiterbildung der Erfindung ist der Festsaug-Sensor mit der Steuereinrichtung verbunden. Die Steuereinrichtung ist dazu eingerichtet, das Signal des Festsaug-Sensors zu erfassen und bei Erkennung eines Festsaugens des Saugers die an der Saugöffnung wirksame Saugleistung zu reduzieren. Hierdurch kann ein festgesaugter Sauger schnell wieder gelöst werden. Ein weiterer Vorteil ist, dass der Sauger kann am tiefsten Punkt im OP-Feld belassen werden kann und zum Lösen nicht zwingend bewegt werden muss, Dies erlaubt ein automatisiertes Saugen ohne Hilfsperson. Hierbei kann insbesondere die an der Saugöffnung wirksame Saugleistung für eine vorbestimmte Zeit abgeschaltet werden. Es ist auch vorteilhaft möglich, die Pumpe für eine vorbestimmte Zeit umgekehrt zu betreiben, so dass ein Überdruck im Sauger erzeugt wird, durch den sich der Sauger wieder lösen lässt.

Gemäß einer vorteilhaften Weiterbildung der Erfindung ist die Steuereinrichtung dazu eingerichtet, in Phasen der Reduzierung oder Abschaltung der an der Saugöffnung wirksamen Saugleistung eine Ventileinrichtung zu betätigen. Die geöffnete Ventileinrichtung verbindet die Saugöffnung des Saugers mit einer Flüssigkeitsquelle, z. B. mit einem Behälter, der physiologische Kochsalzlösung enthält. Die Ventileinrichtung kann z. B. als Relais-gesteuerte Quetschklemme oder als Elektromagnetventil ausgebildet sein. Hierdurch wird ein geschlossenes System geschaffen, durch das Verunreinigungen von außen vermieden werden können. Auch ein unerwünschtes Eindringen von Luft in das System der Absaugvorrichtung kann hiermit vermieden werden.

Gemäß einer vorteilhaften Weiterbildung der Erfindung ist der Festsaug-Sensor als Drucksensor ausgebildet, der derart mit dem Sauger, der Saugseite der Pumpe oder einer Verbindung dazwischen verbunden ist, dass der von der Pumpe erzeugte Unterdruck erfassbar ist. Die Steuereinrichtung ist dazu eingerichtet, den vom Drucksensor erfassten Druck auszuwerten und bei Unterschreitung eines vorbestimmten Druckwertes und/oder bei Erkennung eines für ein Festsaugen typischen Zeitverlaufs des Drucks ein Festsaugen des Saugers zu erkennen. Der vorbestimmte Druckwert kann z. B. 300 mm Hg sein, d. h. ein Unterdruck von 2,25 Pa.

Gemäß einer vorteilhaften Weiterbildung der Erfindung ist der Festsaug-Sensor als Stromsensor ausgebildet, der die Stromaufnahme einer zum Antrieb der Pumpe vorgesehenen Elektromotoreinrichtung erfasst. Die Steuereinrichtung ist dazu eingerichtet, den vom Stromsensor erfassten Strom auszuwerten und bei Überschreitung eines vorbestimmten Stromwertes und/oder bei Erkennung eines für ein Festsaugen typischen Zeitverlaufs des Stroms ein Festsaugen des Saugers zu erkennen. Der vorbestimmte Stromwert bzw. der typische Zeitverlauf des Stroms ist dabei in Abhängigkeit der elektrischen Charakteristika der verwendeten Elektromotoreinrichtung festzulegen.

Allen zuvor genannten Ausgestaltungen der Erfindung ist gemein, dass die Veränderung bzw. die Reduzierung der an der Saugöffnung wirksamen Saugleistung auf unterschiedliche Arten erfolgen kann. Eine Möglichkeit besteht darin, dass die wirksame Pumpleistung der Pumpe beeinflusst wird, z. B. durch entsprechende Anpassung der Betriebsspannung eines für den Antrieb der Pumpe vorgesehenen Elektromotors. Eine weitere Möglichkeit besteht darin, dass ein Bypass zugeschaltet wird, z. B. in der Art, dass der Eingang der Pumpe ganz oder vollständig mit dem Ausgang der Pumpe verbunden wird. Schließlich besteht eine weitere Möglichkeit darin, den Sauger oder eine Verbindung des Saugers mit der Pumpe, z. B. einen Schlauch, ganz oder teilweise abzusperren, z. B. durch ein elektrisch betätigbares Ventil. Ebenso ist es möglich, dass die Saugöffnung hinsichtlich ihres Querschnitts verändert oder geschlossen wird, z. B. durch eine automatisch betätigbare Verschlussklappe. Auch Kombinationen der zuvor genannten Möglichkeiten zur Veränderung bzw. der Reduzierung der an der Saugöffnung wirksamen Saugleistung können vorteilhaft realisiert werden.

Neben den zuvor erläuterten medizinischen Anwendungen ist die Erfindung in sämtlichen Ausgestaltungen auch für andere medizinische Einsatzfälle vorteilhaft verwendbar, wie z. B. im Bereich der Zahnmedizin. Neben dem Absaugen von Blut und Körperflüssigkeiten kann die Absaugvorrichtung auch zum Absaugen von Spülflüssigkeit verwendet werden.

Gemäß einer vorteilhaften Weiterbildung der Erfindung ist die Absaugvorrichtung der zuvor beschriebenen Art als medizinische Absaugvorrichtung ausgebildet. Die medizinische Absaugvorrichtung ist insbesondere für die Absaugung von Blut und/oder anderen Körperflüssigkeiten während eines chirurgischen Eingriffs eingerichtet.

Vorteilhaft kann die Absaugvorrichtung auch im nicht-medizinischen Bereich eingesetzt werden, z. B. als Schmutzwasser-Absaugeinrichtung, Nasssauger, Tauchpumpe oder Feuerwehrpumpe. Die Absaugvorrichtung kann auch zum Absaugen von Öl beim Ölwechsel eines Autos, oder entsprechend zur Absaugung anderer Flüssigkeiten eines Autos, verwendet werden. Bei einer Anwendung als Schmutzwasser-Absaugvorrichtung (Schmutzwasserpumpe) kann z. B. ein Verstopfen des Saugers durch größere, feste Bestandteile im Wasser auftreten, d. h. durch Schmutzpartikel. Diese Bestandteile können ein Festsaugen verursachen, was zu einem unerwünschten Stoppen oder Drosseln der Saugleistung führen würde. Auch an dieser Stelle kann durch die Erfindung Abhilfe geschaffen werden. Vergleichbares gilt für Nasssauger und Tauchpumpen, die z. B. unerwünscht Umgebungsluft oder Schmutz ansaugen können. Die hierbei auftretenden unerwünschten Effekte können durch die Erfindung vermieden oder zumindest deutlich verringert werden. Im Falle von Feuerwehrpumpen können vergleichbare Probleme z. B. beim Saugen von Löschwasser aus sehr verschmutzten Löschteichen auftreten.

Die Erfindung wird nachfolgend anhand eines Ausführungsbeispiels unter Verwendung von Zeichnungen näher erläutert. Es zeigen:
- Figur 1 -: eine medizinische Absaugvorrichtung in schematischer Darstellung und
- Figur 2 -: eine Steuereinrichtung in schematischer Darstellung und
- Figur 3 -: eine Steuercharakteristik im Hinblick auf die an der Saugöffnung wirksame Saugleistung.

In den Figuren werden gleiche Bezugszeichen für einander entsprechende Elemente verwendet.

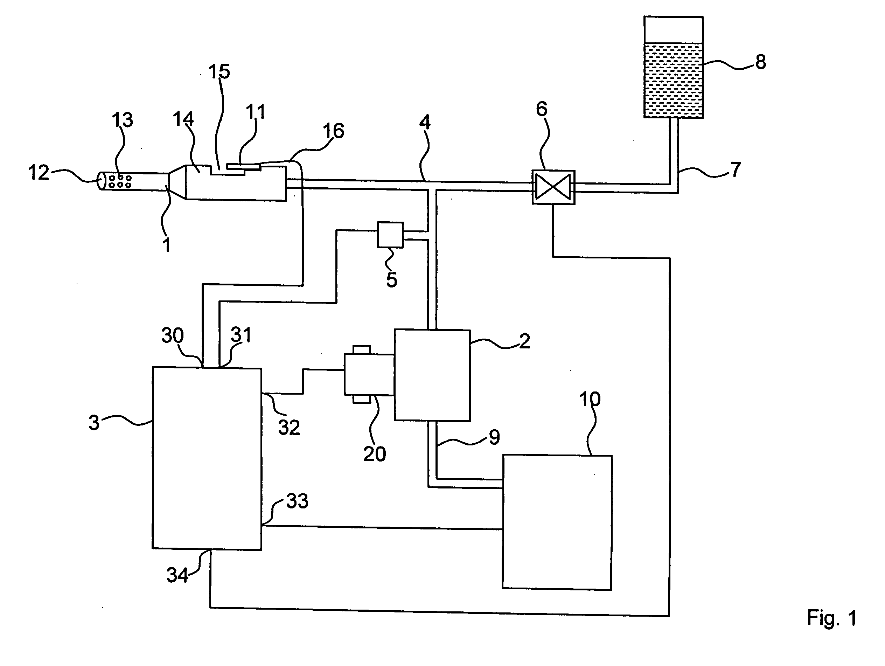

Die Figur 1 zeigt als Teile einer medizinischen Absaugvorrichtung einen Sauger 1, eine Pumpe 2, eine Steuereinrichtung 3 und einen Drucksensor 5. Mit der medizinischen Absaugvorrichtung sind ferner eine Ventileinrichtung 6, ein Vorratsbehälter 8 mit physiologischer Kochsalzlösung sowie eine Herz-Lungen-Maschine 10 verbunden. Der Sauger 1, die Ventileinrichtung 6, der Drucksensor 5 sowie eine Saugseite der Pumpe 2 sind untereinander mit einer Schlauchanordnung 4 verbunden, die unter anderem eine Verbindung zwischen dem Sauger 1 und der Pumpe 2 herstellt. Die Ventileinrichtung 6 ist auf ihrer anderen Seite über einen Schlauch 7 mit dem Vorratsbehälter 8 verbunden. Ferner ist eine Druckseite der Pumpe 2 über einen Schlauch 9 mit der Herz-Lungen-Maschine 10 verbunden.

Die Pumpe 2 weist einen Elektromotor 20 zum Antrieb der Pumpe auf. Bei laufendem Elektromotor 20 erzeugt die Pumpe 2 an ihrer mit der Schlauchanordnung 4 verbundenen Saugseite einen Unterdruck, durch den über den Sauger 1 durch die Schlauchanordnung 4 Körperflüssigkeiten ansaugbar sind. Die angesaugten Körperflüssigkeiten werden aus der Pumpe 2 auf der Druckseite durch den Schlauch 9 abgegeben und der Herz-Lungen-Maschine 10 zugeführt. In der Herz-Lungen-Maschine 10 erfolgt eine Aufbereitung der zugeführten Körperflüssigkeit durch eine in die Herz-Lungen-Maschine 10 integrierte Blutverarbeitungseinrichtung. Die Blutverarbeitungseinrichtung weist ein besonders geartetes Filter auf, durch das ein Volumenanteil des Blutes in einem in die Herz-Lungen-Maschine 10 integrierten Sammelbecken vorübergehend zwischengelagert wird.

Der Sauger 1 weist einen Griffbereich 14 auf, der zum manuellen Greifen des Saugers 1 ausgebildet ist. Vom Griffbereich 14 aus erstreckt sich ein länglicher schmaler Saugtubus, der am distalen Ende eine frontseitige Saugöffnung 12 sowie mehrere seitlich angeordnete Saugöffnungen 13 aufweist. Der Sauger 1 weist im Griffbereich 14 eine Aussparung 15 auf, die z. B. abgestuft ausgebildet sein kann. In die Aussparung 15 ist ein piezoelektrischer Sensor 11 eingebettet, der als Schallwellensensor dient. Der piezoelektrische Sensor 11 ist nur etwa auf der halben Länge seiner Längserstreckung an dem Griffbereich 14 befestigt, der übrige Teil ist freischwingend. Der piezoelektrische Sensor 11 ist über eine elektrische Leitung 16 mit der Steuereinrichtung 3 verbunden.

Die Steuereinrichtung 3 kann z. B. als elektronisches Steuergerät ausgebildet sein. Die Steuereinrichtung 3, deren innerer Aufbau im Weiteren noch erläutert wird, weist einen Schallwellensensor-Eingangsanschluss 30 auf, der mit dem piezoelektrischen Sensor 11 verbunden ist. Ferner weist die Steuereinrichtung 3 einen Drucksensor-Eingangsanschluss 31 auf, der über eine elektrische Leitung mit dem Drucksensor 5 verbunden ist. Die Steuereinrichtung 3 weist außerdem einen Pumpensteuerungs-Ausgangsanschluss 32 auf, der über eine elektrische Leitung mit dem Elektromotor 20 der Pumpe 2 verbunden ist. Die Steuereinrichtung 3 weist einen Hämolyse-Punktzahl-Ausgabeanschluss 33 auf, der über eine elektrische Leitung mit der Herz-Lungen-Maschine 10 verbunden ist. Zudem weist die Steuereinrichtung 3 einen Ventileinrichtungs-Ausgangsanschluss 34 auf, der über eine elektrische Leitung mit der Ventileinrichtung 6 verbunden ist. Statt der elektrischen Leitungen können auch drahtlose Verbindungen vorgesehen sein, z.B. Funkverbindungen.

Über den Schallwellensensor-Eingangsanschluss 30 empfängt die Steuereinrichtung 3 die Signale des piezoelektrischen Sensors 11. Über den Drucksensor-Eingangsanschluss 31 empfängt die Steuereinrichtung 3 die Drucksignale des Drucksensors 5. Über den Pumpensteuerungs-Ausgangsanschluss 32 steuert die Steuereinrichtung 3 die Pumpleistung der Pumpe 2 und hiermit die an der Saugöffnung 12, 13 wirksame Saugleistung. Die Steuerung der Pumpe 2 kann durch Beeinflussung der Betriebsspannung des Elektromotors 20 oder durch Steuerung der Drehzahl der Pumpe 2 erfolgen.

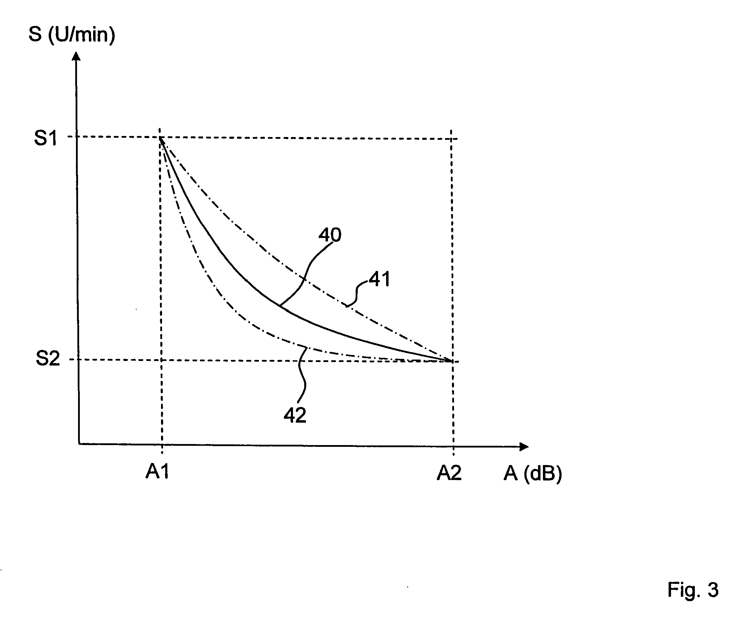

Die Steuereinrichtung 3 wertet die von dem piezoelektrischen Sensor 11 empfangenen Signale aus und berechnet hieraus die Hämolyse-Punktzahl. Zudem bestimmt die Steuereinrichtung 3 hieraus eine Solldrehzahl für die Pumpe 2, die zur Ansteuerung des Elektromotors 20 verwendet wird. Hierdurch wird das unerwünschte Schlürfen des Saugers 1 vermieden. Die Steuercharakteristik wird nachfolgend noch anhand der Figur 3 erläutert.

Über den Hämolyse-Punktzahl-Ausgabeanschluss 33 übersendet die Steuereinrichtung 3 jeweils aktuelle Daten für die Hämolyse-Punktzahl an die Herz-Lungen-Maschine 10, die diese Daten verarbeitet und entsprechend in den Prozess der Aufbereitung des Blutes einfließen lässt. Über den Ventileinrichtungs-Ausgangsanschluss 34 steuert die Steuereinrichtung 3 die Ventileinrichtung 6 über ein elektrisches Signal. Die Ventileinrichtung 6 kann hierfür z. B. als Elektromagnetventil oder als Relais-gesteuerte Quetschklemme ausgebildet sein. Die Ventileinrichtung 6 kann durch das Signal von der Steuereinrichtung 3 geöffnet oder geschlossen werden. In geöffnetem Zustand fließt Flüssigkeit aus dem Vorratsbehälter 8 durch den Schlauch 7 in die Schlauchanordnung 4 und hierdurch durch den Sauger 1 zu den Saugöffnungen 12, 13. In geschlossenem Zustand der Ventileinrichtung 6 ist die Verbindung zwischen dem Schlauch 7 und der Schlauchanordnung 4 abgesperrt.

Die die Schlauchanordnung 4 kann auch mit der Herz-Lungen-Maschine 10 verbunden sein. In diesem Fall kann auch zwischengelagertes Blut aus dem Sammelbecken der Herz-Lungen-Maschine 10 bzw. aus dem venösen System der Herz-Lungen-Maschine 10 zum Druckausgleich in die Schlauchanordnung 4 eingespeist werden verwendet werden, d.h. es wird eine passager geöffnete Kurzschlussverbindung hergestellt.

Die Steuereinrichtung 3 prüft zudem die vom Drucksensor 5 gelieferten Druckwerte. Sofern die Druckwerte einen Unterdruck von 2,25 mbar unterschreiten, stoppt die Steuereinrichtung 3 die Pumpe 2 und öffnet die Ventileinrichtung 6. Nach Ablauf einer vorbestimmten Zeit oder mit dem Erreichen eines passenden Druckniveaus schließt die Steuereinrichtung 3 die Ventileinrichtung 6 wieder und schaltet die Pumpe 2 wiederum ein. Hierdurch kann das Festsaugen des Saugers 1 aufgehoben werden.

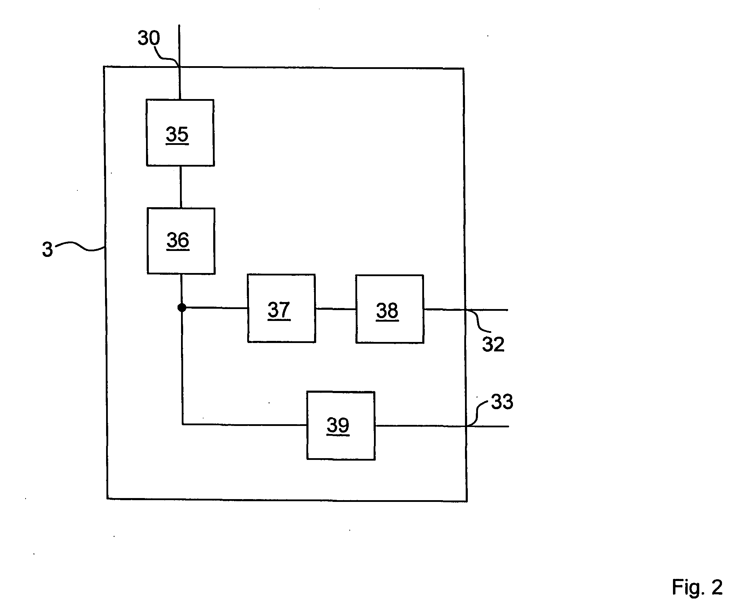

Die Figur 2 zeigt beispielhaft einen inneren Aufbau der Steuereinrichtung 3 mit weiteren Details. Das am Schallwellensensor-Eingangsanschluss 30 empfangene Signal wird einem Vorverstärker 35 zugeführt. Vorteilhaft wird hier ein relativ hochohmiger Vorverstärker mit einer Eingangsimpedanz von wenigstens 10 Megaohm verwendet. Das Ausgangssignal des Vorverstärkers 35 wird einem Hochpass-Filter 36 mit einer Grenzfrequenz von 16 kHz zugeführt. Das Ausgangssignal des Hochpass-Filters 36 enthält daher nur Frequenzanteile oberhalb von 16 kHz. Dieses Ausgangssignal wird einem Noise-Gate 37 zugeführt. Das Noise-Gate 37 hat die Funktion, eine einstellbare Verzögerung bis zu einem Ansprechen zu realisieren. Zudem beinhaltet das Noise-Gate 37 die Funktion einer einstellbaren Ansprechschwelle, d.h. einer Mindestamplitude der Schallwellen. Hierdurch sind als Steuersignal eine Mindestzeit und Mindestintensität der Eingangssignale des Noise-Gate 37 einstellbar, so dass festlegbar ist, ab welcher Schallwellenintensität und -dauer eine Beeinflussung der Saugleistung erfolgen soll. Somit wird der Einfluss der Signale des Schallwellensensors 11 auf die Funktion der Pumpe 2 auf Signale beschränkt, die für eine gewisse Mindestzeit und mit einer gewissen Mindestintensität vorhanden sind, so dass kurzzeitige Signale oder Signale mit geringer Intensität keine unerwünschte Veränderung in der Saugleistung der Pumpe 2 bewirken. Das Ausgangssignal des Noise-Gate 37 wird einem Verstärker 38 mit einstellbarer Verstärkung zugeführt. Das Ausgangssignal des Verstärkers 38 wird sodann über den Pumpensteuerungs-Ausgangsanschluss 32 dem Elektromotor 20 der Pumpe 2 zugeführt. Der Verstärker 38 hat hierbei vorteilhaft eine invertierende Charakteristik, wie nachfolgend noch anhand der Figur 3 beschrieben.

Das Ausgangssignal des Hochpass-Filters 36 wird zudem einen Mikroprozessor 39 zugeführt. Der Mikroprozessor 39 berechnet aus den über die Zeit von dem Hochpass-Filter 36 abgegebenen Signalamplituden Zeitintegral. Der jeweils aktuelle Wert des Zeitintegrals wird vom Mikroprozessor 39 über den Hämolyse-Punktzahl-Ausgabeanschluss 33 ausgegeben.

Die Figur 3 zeigt vorteilhafte Steuercharakteristika der Steuereinrichtung 3. Hierbei ist an der Abszisse die Amplitude A des Ausgangssignals des Hochpass-Filters 36 dargestellt, z. B. in der Einheit Dezibel (dB). An der Ordinate ist die einzustellende Drehzahl S der Pumpe 2 dargestellt, z. B. in der Einheit Umdrehungen/Minute (U/min). Erkennbar ist, dass eine Beeinflussung der Pumpe 2 erst erfolgt, wenn die Amplitude A den Wert A1 überschreitet. Bei kleineren Amplituden wird durch die Steuereinrichtung 3 die Drehzahl der Pumpe 2 nicht beeinflusst. Bei einer Überschreitung des unteren Grenzwertes A1 wird die Drehzahl S zwischen einem oberen Wert S1 und einem unteren Wert S2 durch die Steuereinrichtung 3 beeinflusst. Hierbei ist eine invertierende Steuercharakteristik vorgesehen, z. B. gemäß dem Verlauf 40. Mit Überschreitung des unteren Grenzwerts A1 und zunehmender Amplitude A wird die Drehzahl S von dem oberen Wert S1 hin zu dem unteren Wert S2 verringert. Der Wert S2 wird bei einem oberen Grenzwert A2 für die Amplitude A erreicht. Je nach Anwendungsfall sind auch andere Steuerkurven sinnvoll, wie z. B. durch die Verläufe 41, 42 dargestellt.

## Patentansprüche

1. Absaugvorrichtung zur Absaugung von Flüssigkeiten, mit einem zur Aufnahme der Flüssigkeit mit wenigstens einer Saugöffnung (12, 13) versehenen Sauger (1) und einer mit dem Sauger (1) verbundenen Pumpe (2), die dazu eingerichtet ist, im Sauger (1) einen Saug-Unterdruck zu erzeugen, wobei eine Steuereinrichtung (3) und wenigstens ein mit der Steuereinrichtung (3) verbundener Sensor (5, 11) vorgesehen ist, wobei die Steuereinrichtung (3) dazu eingerichtet ist, in Abhängigkeit von den von dem Sensor (5, 11) aufgenommenen Signalen die an der Saugöffnung (12, 13) wirksame Saugleistung zu beeinflussen, wobei der Sensor (5, 11) als Schallwellensensor (11) ausgebildet ist, der dazu eingerichtet ist, durch den Sauger (1) bei dessen Betrieb erzeugte Schallwellen aufzunehmen, **dadurch gekennzeichnet, dass** das vom Schallwellensensor (11) abgegebene Signal einem Noise-Gate (37) zugeführt ist, das die Funktion einer einstellbaren Verzögerung und einer einstellbaren Ansprechschwelle bis zum Ansprechen aufweist, so dass festlegbar ist, ab welcher Schallwellenintensität und Schallwellendauer eine Beeinflussung der wirksamen Saugleistung erfolgt.

2. Absaugvorrichtung nach Anspruch 1, **dadurch gekennzeichnet, dass** die Steuereinrichtung (3) und/oder der Schallwellensensor (11) dazu eingerichtet sind, anhand der aufgenommenen Schallwellen wenigstens ein vorbestimmtes charakteristisches Schallwellenmuster zu detektieren und bei Detektion eines solchen charakteristischen Schallwellenmusters die an der Saugöffnung (12, 13) wirksame Saugleistung zu verändern.

3. Absaugvorrichtung nach Anspruch 2, **dadurch gekennzeichnet, dass** die Steuereinrichtung (3) dazu eingerichtet ist, bei Detektion eines solchen charakteristischen Schallwellenmusters die an der Saugöffnung (12, 13) wirksame Saugleistung zu reduzieren.

4. Absaugvorrichtung nach einem der vorhergehenden Ansprüche, **dadurch gekennzeichnet, dass** der Schallwellensensor (11) am Sauger (1), an der Saugseite der Pumpe (2), einer Verbindung (4) zwischen dem Sauger (1) und der Pumpe (2) oder im Sauger (1), der Pumpe (2) oder der Verbindung (4) dazwischen angeordnet ist.

5. Absaugvorrichtung nach einem der vorhergehenden Ansprüche, **dadurch gekennzeichnet, dass** als charakteristische Schallwellenmuster Schallwellen im Frequenzbereich oberhalb von 11 kHz, insbesondere oberhalb von 15 kHz, detektiert werden.

6. Absaugvorrichtung nach einem der vorhergehenden Ansprüche, **dadurch gekennzeichnet, dass** als charakteristische Schallwellenmuster Schallwellen in einem Frequenzbereich bis maximal 50 kHz detektiert werden.

7. Absaugvorrichtung nach einem der vorhergehenden Ansprüche, **dadurch gekennzeichnet, dass** der Schallwellensensor (11) als piezoelektrischer Sensor ausgebildet ist, der am Sauger (1) befestigt ist.

8. Absaugvorrichtung nach Anspruch 7, **dadurch gekennzeichnet, dass** der piezoelektrische Sensor (11) wenigstens zum Teil freischwingend am Sauger (1) befestigt ist.

9. Absaugvorrichtung nach einem der vorhergehenden Ansprüche, **dadurch gekennzeichnet, dass** wenigstens ein Festsaug-Sensor (5) zum Erfassen eines Festsaugens des Saugers (1) vorgesehen ist, der mit dem Sauger (1), der Pumpe (2) oder einer Verbindung (4) dazwischen verbunden ist.

10. Absaugvorrichtung nach Anspruch 9, **dadurch gekennzeichnet, dass** der Festsaug-Sensor (5) mit der Steuereinrichtung (3) verbunden ist, wobei die Steuereinrichtung (3) dazu eingerichtet ist, das Signal des Festsaug-Sensors (5) zu erfassen und bei Erkennung eines Festsaugens des Saugers (1) die an der Saugöffnung (12, 13) wirksame Saugleistung zu reduzieren.

11. Absaugvorrichtung nach Anspruch 10, **dadurch gekennzeichnet, dass** die Steuereinrichtung (3) dazu eingerichtet ist, in Phasen der Reduzierung oder Abschaltung der an der Saugöffnung (12, 13) wirksamen Saugleistung eine Ventileinrichtung (6) zu betätigen, wobei die geöffnete Ventileinrichtung (6) die Saugöffnung (12, 13) des Saugers (1) mit einer Flüssigkeitsquelle (8) verbindet.

12. Absaugvorrichtung nach einem der Ansprüche 9 bis 11, **dadurch gekennzeichnet, dass** der Festsaug-Sensor (5) als Drucksensor ausgebildet ist, der derart mit dem Sauger (1), der Saugseite der Pumpe (2) oder einer Verbindung (4) dazwischen verbunden ist, dass der von der Pumpe (2) erzeugte Unterdruck erfassbar ist, und die Steuereinrichtung (3) dazu eingerichtet ist, den vom Drucksensor (5) erfassten Druck auszuwerten und bei Unterschreitung eines vorbestimmten Druckwerts und/oder bei Erkennung eines für ein Festsaugen typischen Zeitverlaufs des Drucks ein Festsaugen des Saugers (1) zu erkennen.

13. Absaugvorrichtung nach einem der Ansprüche 9 bis 12, **dadurch gekennzeichnet, dass** Festsaug-Sensor (5) als Stromsensor ausgebildet ist, der die Stromaufnahme einer zum Antrieb der Pumpe (2) vorgesehenen Elektromotoreinrichtung (20) erfasst, und die Steuereinrichtung (3) dazu eingerichtet ist, den vom Stromsensor erfassten Strom auszuwerten und bei Überschreitung eines vorbestimmten Stromwerts und/oder bei Erkennung eines für ein Festsaugen typischen Zeitverlaufs des Stroms ein Festsaugen des Saugers (1) zu erkennen.

14. Absaugvorrichtung nach einem der vorhergehenden Ansprüche, **dadurch gekennzeichnet, dass** die Veränderung bzw. die Reduzierung der an der Saugöffnung (12, 13) wirksamen Saugleistung durch Beeinflussung der wirksamen Pumpleistung der Pumpe (2), durch Hinzuschaltung eines Bypasses, durch zumindest teilweises Absperren des Saugers (1) oder einer Verbindung (4) des Saugers (1) mit der Pumpe (2) und/oder durch Verändern oder Schließen der Saugöffnung (12, 13) erfolgt.

15. Als medizinische Absaugvorrichtung ausgebildete Absaugvorrichtung nach einem der vorhergehenden Ansprüche.

## Claims

1. A suction device for suctioning liquids, comprising a suction element (1), which is provided with at least one suction opening (12, 13) for receiving the liquid, and a pump (2) which is connected to the suction element (1) and which is equipped to generate a suction vacuum in the suction element (1), wherein a control device (3) and at least one sensor (5, 11) which is connected to the control device (3) are provided, wherein the control device (3) is arranged to influence the power acting at the suction opening (12, 13) dependent on the signal received from the sensor (5, 11), wherein the sensor (5, 11) is designed as an acoustic wave sensor (11) which is arranged to detect acoustic waves which are generated by the suction element (1) during the operation thereof, the output signal of the acustic wave sensor (11) is fed to a noise gate (37), which has the function of providing an adjustable delay and of an adjustable response threshold until a response, such that it is possible to establish the acoustic wave intensity and acoustic wave duration starting from which the suction power is to be influenced.

2. The suction device as claimed in claim 1, **characterized in that** the control device (3) and/or the acoustic wave sensor (11) are equipped to detect at least one predetermined characteristic acoustic wave pattern on the basis of the detected acoustic waves and, when such a characteristic acoustic wave pattern is detected, to change the suction power acting at the suction opening (12, 13).

3. The suction device as claimed in claim 2, **characterized in that** the control device (3) is equipped, upon detection of such a characteristic acoustic wave pattern, to reduce the suction power acting at the suction opening (12, 13).

4. The suction device as claimed in one of the preceding claims, **characterized in that** the acoustic wave sensor (11) is arranged on the suction element (1), on the suction side of the pump (2), on a connection (4) between the suction element (1) and the pump (2), or in the suction element (1), in the pump (2) or in the connection (4) therebetween.

5. The suction device as claimed in one of the preceding claims, **characterized in that** acoustic waves in the frequency range above 11 kHz, in particular above 15 kHz, are detected as characteristic acoustic wave patterns.

6. The suction device as claimed in one of the preceding claims, **characterized in that** acoustic waves in a frequency range of up to at most 50 kHz are detected as characterized acoustic wave patterns.

7. The suction device as claimed in one of the preceding claims, **characterized in that** the acoustic wave sensor (11) is designed as a piezoelectric sensor, which is mounted on the suction element (1).

8. The suction device as claimed in claim 7, **characterized in that** the piezoelectric sensor (11) is mounted on the suction element (1) so as to be freely oscillating at least in part.

9. The suction device as claimed in one of the preceding claims, **characterized in that** at least one adherence-suction sensor (5) is provided which is connected to the suction element (1), to the pump (2) or to a connection (4) therebetween, in such a way that an adherence suction of the suction element (1) can be registered.

10. The suction device as claimed in claim 9, **characterized in that** the adherence-suction sensor (5) is connected to the control device (3), wherein the control device (3) is equipped to register the signal from the adherence-suction sensor (5) and, upon detection of an adherence suction of the suction element (1), to reduce the suction power acting at the suction opening (12, 13).

11. The suction device as claimed in claim 10, **characterized in that** the control device (3) is equipped to actuate a valve device (6) in phases of the reduction or switching-off of the suction power acting at the suction opening (12, 13), wherein the opened valve device (6) connects the suction opening (12, 13) of the suction element (1) to a liquid source (8).

12. The suction device as claimed in one of claims 9 through 11, **characterized in that** the adherence-suction sensor (5) is designed as a pressure sensor which is connected to the suction element (1), to the suction side of the pump (2) or to a connection (4) therebetween, in such a way that the vacuum generated by the pump (2) can be registered, and the control device (3) is arranged to evaluate the pressure registered by the pressure sensor (5) and to detect an adherence suction of the suction element (1) when a predetermined pressure value is not reached and/or when a time profile of the pressure typical of an adherence suction is detected.

13. The suction device as claimed in one of claims 9 through 12, **characterized in that** the adherence-suction sensor (5) is designed as a current sensor which detects the current consumption of an electric motor device (20) provided for driving the pump (2), and the control device (3) is arranged to evaluate the current registered by the current sensor and to detect an adherence suction of the suction element (1) when a predetermined current value is exceeded and/or when a time profile of the current typical of an adherence suction is detected.

14. The suction device as claimed in one of the preceding claims, **characterized in that** the change or the reduction of the suction power acting at the suction opening (12, 13) is effected by influencing the actual pumping power of the pump (2), by switching in a bypass, by at least partially shutting off the suction element (1) or a connection (4) of the suction element (1) to the pump (2), and/or by changing or closing the suction opening (12, 13).

15. The suction device as claimed in one of the preceding claims designed as a medical suction device.

## Revendications

1. Ensemble d'aspiration de liquides présentant
un aspirateur (1) doté d'au moins une ouverture d'aspiration (12, 13) pour la reprise du liquide et une pompe (2) raccordée à l'aspirateur (1) et conçue pour former une dépression d'aspiration dans l'aspirateur (1),
un dispositif de commande (3) et au moins un capteur (5, 11) raccordé au dispositif de commande (3) étant prévus,
le dispositif de commande (3) étant conçu pour agir sur la puissance d'aspiration agissant sur l'ouverture d'aspiration (12, 13) en fonction des signaux enregistrés par le capteur (5, 11),
le capteur (5, 11) étant configuré comme capteur (11) d'ondes acoustiques conçu pour enregistrer les ondes acoustiques produites par l'aspirateur (1) lors de son fonctionnement,
**caractérisé en ce que**
le signal délivré par le capteur (11) d'ondes acoustiques est apporté à un port (37) de bruit qui présente une fonction de ralentissement ajustable et un seuil ajustable de réponse de manière à pouvoir définir à partir de quelle intensité de l'onde acoustique et de quelle durée de l'onde acoustique il faut agir sur la puissance d'aspiration active.

2. Ensemble d'aspiration selon la revendication 1, **caractérisé en ce que** le dispositif de commande (3) et/ou le capteur (11) d'ondes acoustiques sont conçus pour, à l'aide des ondes acoustiques enregistrées, détecter au moins un motif prédéterminé et caractéristique des ondes acoustiques et, en cas de détection de ce motif caractéristique des ondes acoustiques, pour modifier la puissance d'aspiration qui agit sur l'ouverture d'aspiration (12, 13).

3. Ensemble d'aspiration selon la revendication 2, **caractérisé en ce que** le dispositif de commande (3) est conçu pour, lors de la détection de ce motif caractéristique des ondes acoustiques, réduire la puissance d'aspiration qui agit sur l'ouverture d'aspiration (12, 13).

4. Ensemble d'aspiration selon l'une des revendications précédentes, **caractérisé en ce que** le capteur (11) d'ondes acoustiques est disposé sur l'aspirateur (1), sur le côté d'aspiration de la pompe (2), sur un conduit (4) qui relie l'aspirateur (1) et la pompe (2) ou dans l'aspirateur (1), la pompe (2) ou le conduit (4), entre eux.

5. Ensemble d'aspiration selon l'une des revendications précédentes, **caractérisé en ce que** le motif caractéristique des ondes acoustiques détecté est constitué d'ondes acoustiques dans la plage de fréquences supérieure à 11 kHz et en particulier au-dessus de 15 kHz.

6. Ensemble d'aspiration selon l'une des revendications précédentes, **caractérisé en ce que** le motif caractéristique des ondes acoustiques détecté est constitué d'ondes acoustiques dans une plage de fréquences atteignant au plus 50 kHz.

7. Ensemble d'aspiration selon l'une des revendications précédentes, **caractérisé en ce que** le capteur (11) d'ondes acoustiques est configuré comme capteur piézoélectrique fixé sur l'aspirateur (1).

8. Ensemble d'aspiration selon la revendication 7, **caractérisé en ce que** le capteur piézoélectrique (11) est fixé sur l'aspirateur (1) de manière à pouvoir au moins en partie osciller librement.

9. Ensemble d'aspiration selon l'une des revendications précédentes, **caractérisé en ce qu'**au moins un capteur (5) de blocage d'aspiration qui détecte le blocage d'aspiration de l'aspirateur (1) et est raccordé à l'aspirateur (1), la pompe (2) ou un conduit (4) qui les relie.

10. Ensemble d'aspiration selon la revendication 9, **caractérisé en ce que** le capteur (5) de blocage d'aspiration est raccordé au dispositif de commande (3), le dispositif de commande (3) étant conçu pour saisir le signal du capteur (5) de blocage d'aspiration et, lors de la détection d'un blocage de l'aspiration de l'aspirateur (1), réduire la capacité d'aspiration qui agit sur l'ouverture d'aspiration (12, 13).

11. Ensemble d'aspiration selon la revendication 10, **caractérisé en ce que** le dispositif de commande (3) est conçu pour actionner un dispositif de soupape (6) dans les phases de réduction ou de débranchement de la puissance d'aspiration qui agit sur l'ouverture d'aspiration (12, 13), le dispositif de soupape (6) ouvert raccordant l'ouverture d'aspiration (12, 13) de l'aspirateur (1) de liquide à une source (8).

12. Ensemble d'aspiration selon l'une des revendications 9 à 11, **caractérisé en ce que** le capteur (5) de blocage d'aspiration est configuré comme capteur de pression qui est raccordé à l'aspirateur (1), au côté d'aspiration de la pompe (2) ou à un conduit (4) qui les relie de telle sorte que la dépression formée par la pompe (2) puisse être saisie, et le dispositif de commande (3) est conçu pour évaluer la pression détectée par le capteur de pression (5) et pour détecter un blocage de l'aspiration de l'aspirateur (1) lorsqu'une valeur de pression prédéterminée n'est pas atteinte et/ou lorsque l'évolution temporelle de la pression typique d'un blocage d'aspiration est détectée.

13. Ensemble d'aspiration selon l'une des revendications 9 à 12, **caractérisé en ce que** le capteur (5) de blocage d'aspiration est configuré comme capteur de courant qui saisit le courant consommé par un dispositif (20) à moteur électrique prévu pour entraîner la pompe (2), le dispositif de commande (3) étant conçu pour évaluer le courant sais par le capteur de courant et détecter un blocage de l'aspiration de l'aspirateur (1) lorsqu'une valeur de courant prédéterminée n'est pas atteinte et/ou lorsqu'une évolution temporelle du courant typique d'un blocage d'aspiration est détectée.

14. Ensemble d'aspiration selon l'une des revendications précédentes, **caractérisé en ce que** la modification ou la réduction de la puissance d'aspiration qui agit sur l'ouverture d'aspiration (12, 13) s'effectue en agissant sur la puissance effective de la pompe (2), par raccordement d'une dérivation, par un blocage au moins partiel de l'aspirateur (1) ou d'un conduit (4) qui relie l'aspirateur (1) à la pompe (2) et/ou par modification ou fermeture de l'ouverture d'aspiration (12, 13).

15. Ensemble d'aspiration selon l'une des revendications précédentes, configuré comme ensemble d'aspiration médical.
